# EUROPEAN PATENT SPECIFICATION

(11) **EP 4 586 185 B1**
(45) Date of publication and mention of the grant of the patent: **12.08.2026**
(21) Application number: 23896013.2
(22) Date of filing: 30.06.2023
(51) Int. Cl.: G06V 20/69, C12Q 1/18, G16H 10/40

(54) **MINIMUM INHIBITORY CONCENTRATION RECOGNITION METHOD, APPARATUS AND DEVICE, AND STORAGE MEDIUM**
VERFAHREN, VORRICHTUNG UND VORRICHTUNG ZUR ERKENNUNG EINER MINIMALEN HEMMKONZENTRATION UND SPEICHERMEDIUM
PROCÉDÉ, APPAREIL ET DISPOSITIF DE RECONNAISSANCE DE CONCENTRATION MINIMALE INHIBITRICE, ET SUPPORT DE STOCKAGE

(30) Priority: 02.12.2022 CN 202211538062
(43) Date of publication of application: 16.07.2025
(73) Proprietor: Autobio Labtec Instruments Co., Ltd., Zhengzhou, Henan 450016 (CN)
(72) Inventor: HOU, Jianping, Zhengzhou, Henan 450016 (CN); WANG, Chao, Zhengzhou, Henan 450016 (CN); ZHANG, Qianqian, Zhengzhou, Henan 450016 (CN); ZHAO, Wanli, Zhengzhou, Henan 450016 (CN); YANG, Liuqing, Zhengzhou, Henan 450016 (CN); LIU, Cong, Zhengzhou, Henan 450016 (CN)
(74) Representative: Kolster Oy Ab
(86) International application number: PCT/CN2023/104579
(87) International publication number: WO 2024/113849

(56) References cited:
- WO-A1-2021/067170
- WO-A1-2022/100387
- WO-A1-2022/109091
- CN-A- 108 090 502
- CN-A- 112 052 868
- CN-A- 115 035 102
- CN-A- 115 829 969
- CN-A- 115 829 969

## Description

The present application claims priority to Chinese Patent Application No.202211538062.9, titled "MINIMUM INHIBITORY CONCENTRATION RECOGNITION METHOD, APPARATUS AND DEVICE, AND STORAGE MEDIUM", filed on December 02, 2022 with the China National Intellectual Property Administration.

### FIELD

The present disclosure relates to the technical field of image processing and computing, and in particular to a method, apparatus and device for determining a minimum inhibitory concentration and a computer-readable storage medium.

### BACKGROUND

The misuse of antibiotics is a significant issue facing humanity in the 21st century. The appropriate use of antibiotics becomes more necessary, and as a result, attention is drawn to accurately and rapidly detecting a minimum antibiotic dosage that effectively acts against bacteria. A minimum inhibitory concentration (MIC) of an antibiotic refers to a minimum antibiotic concentration that can effectively kill bacteria or inhibit the growth of bacteria.

In the conventional technology, the detection of the minimum inhibitory concentration of drugs is generally performed by using a dilution method or by an automated instrument detection method. However, both of the two methods have some drawbacks. For example, an operation process of the broth dilution method takes at least 24 to 48 hours, thus being unfavorable for clinical use; and the automated instrument shortens a reporting time period, but still takes approximately 10 to 12 hours, failing to meet the requirement of rapid diagnosis in clinical emergency and being costly.

WO2022/109091A1 and WO2021/067170A1 represent relevant prior art.

Therefore, attention is drawn to how to address the problem of rapidly and accurately performing automatic detection of the minimum inhibitory concentration of the antibiotic and reducing the detection cost.

### SUMMARY

The objective of the present disclosure is to provide a method, apparatus, and device for determining a minimum inhibitory concentration, and a computer-readable storage medium, to rapidly and accurately implement automatic detection and determination of the minimum inhibitory concentration of an antibiotic and reduce the detection cost.

To address the above technical problem, a method for determining a minimum inhibitory concentration is provided according to the present disclosure. The method includes:
obtaining testing images of a to-be-tested bacterial strain, where the testing images include images of mixed solutions of a to-be-tested antibiotic with different preset gradient concentrations and the to-be-tested bacterial strain and are obtained at a time instant of completing bacteria inhibition;
obtaining test control images of the to-be-tested bacterial strain;
calculating a similarity between the testing images and the test control images corresponding to the testing images, to obtain respective similarity test results of the testing images, where each of the similarity test results indicates similarity or dissimilarity; and
determining the minimum inhibitory concentration of the to-be-tested antibiotic corresponding to the to-be-tested bacterial strain based on the similarity test results and the preset gradient concentrations.

In an embodiment, the test control image includes a preset control image of the to-be-tested bacterial strain and/or images of the mixed solutions of the to-be-tested antibiotic with the different preset gradient concentrations and the to-be-tested bacterial strain and that are obtained at a time instant of not completing bacteria inhibition, where time instant of not completing bacteria inhibition precedes the time instant of completing bacteria inhibition.

In an embodiment, in response to the test control images including images of the mixed solutions of the to-be-tested antibiotic with the different preset gradient concentrations and the to-be-tested bacterial strain and that are obtained at the time instant of not completing bacteria inhibition, the calculating a similarity between the testing images and the test control images corresponding to the testing images, to obtain respective similarity test results of the testing images includes:
calculating the similarity between the testing images and the test control images corresponding to the preset gradient concentrations to obtain the respective similarity test results of the testing images.

In an embodiment, the obtaining testing images of a to-be-tested bacterial strain includes:
performing lensless imaging on each of microwells of an antimicrobial susceptibility test plate after a preset incubation time period following adding a bacterial suspension of the to-be-tested bacterial strain to the microwells, to obtain an original testing image of the to-be-tested bacterial strain, where the microwells are provided with the to-be-tested antibiotic with the preset gradient concentrations in one-to-one correspondence;
identifying microwell regions in original testing images; and
extracting a target region of interest with a preset size from each of the microwell regions, where an image of the target region of interest serves as the testing image.

In an embodiment, the minimum inhibitory concentration is any one of the preset gradient concentrations.

In an embodiment, the determining the minimum inhibitory concentration of the to-be-tested antibiotic corresponding to the to-be-tested bacterial strain based on the similarity test results and the preset gradient concentrations includes:
determining, in response to the similarity test results of the testing images including similarity and dissimilarity, a minimum preset gradient concentration of the preset gradient concentrations corresponding to testing images with similarity test results indicating dissimilarity as the minimum inhibitory concentration.

In an embodiment, the calculating a similarity between the testing images and the test control images corresponding to the testing images, to obtain respective similarity test results of the testing images includes:
calculating the similarity between the testing images and the test control images corresponding to the testing images by using a preset Siamese neural network model to obtain the respective similarity test results of the testing images.

In an embodiment, sister networks in the preset Siamese neural network model are implemented by a feature extraction layer using MobileNet-V2.

In an embodiment, in response to the test control images including the preset control image of the to-be-tested bacterial strain, the calculating the similarity between the testing images and the test control images corresponding to the testing images by using a preset Siamese neural network model to obtain the respective similarity test results of the testing images includes:
extracting dimensionality-reduced features of the testing images and a dimensionality-reduced feature of the preset control image using the preset Siamese neural network model;
for each of the testing images, calculating an Euclidean distance between the dimensionality-reduced feature of the testing image and the dimensionality-reduced feature of the preset control image; and
determining the respective similarity test results of the testing images based on the Euclidean distances and a preset distance threshold.

In an embodiment, the method for determining the minimum inhibitory concentration further includes a training process of the preset Siamese neural network model. The training process of the preset Siamese neural network model includes:
obtaining a training sample set and a training loss function, where the training sample set includes a preset number of pairs of training images and image similarity labels corresponding to the preset number of pairs of training images, each of the image similarity labels indicates similarity or dissimilarity, and the training loss function is expressed as $L \left({x}_{0}, {x}_{1}, y\right) = y \left‖f\left({x}_{0}\right)-f\left({x}_{1}\right)\right‖ + \left(1-y\right) max \left(0, \left(m-\left‖f\left({x}_{0}\right)-f\left({x}_{1}\right)\right‖\right)\right) ,$
where *x*₀ and *x*₁ represent two training images in any one pair of the preset number of pairs of training images, *f*(*x*₀) represents a dimensionality-reduced feature of *x*₀ that is extracted by the model, *f*(*x*₁) represents a dimensionality-reduced feature of *x*₁ that is extracted by the model, *y* represents an image similarity label between *x*₀ and *x*₁, m represents the preset distance threshold, and ∥f(*x*₀)*-f*(*x*₁)∥ represents a square of an Euclidean distance between *f*(*x*₀) and *f*(*x*₁); and
performing iterative training on an initial Siamese neural network model based on the training sample set and the training loss function to obtain the preset Siamese neural network model.

An apparatus for determining a minimum inhibitory concentration is provided according to the present disclosure. The apparatus includes: a first obtaining module, a second obtaining module, a similarity calculation module, and a concentration determination module.

The first obtaining module is configured to obtain testing images of a to-be-tested bacterial strain, where the testing images include images of mixed solutions of a to-be-tested antibiotic with different preset gradient concentrations and the to-be-tested bacterial strain and are obtained at a time instant of completing bacteria inhibition.

The second obtaining module is configured to obtain test control images of the to-be-tested bacterial strain.

The similarity calculation module is configured to calculate a similarity between the testing images and the test control images corresponding to the testing images, to obtain respective similarity test results of the testing images, where each of the similarity test results indicates similarity or dissimilarity.

The concentration determination module is configured to determine the minimum inhibitory concentration of the to-be-tested antibiotic corresponding to the to-be-tested bacterial strain based on the similarity test results and the preset gradient concentrations.

A device for determining a minimum inhibitory concentration is provided according to the present disclosure. The device includes a memory and a processor.

The memory stores a computer program;

The processor is configured to, when executing the computer program, implement the method for determining the minimum inhibitory concentration described above.

Furthermore, a computer-readable storage medium is provided according to the present disclosure. The computer-readable storage medium stores a computer program. The computer program, when being executed by a processor, implements the method for determining a minimum inhibitory concentration described above.

The method for determining the minimum inhibitory concentration according to the present disclosure includes: obtaining testing images of a to-be-tested bacterial strain, where the testing images include images of mixed solutions of a to-be-tested antibiotic with different preset gradient concentrations and the to-be-tested bacterial strain and are obtained at a time instant of completing bacteria inhibition; obtaining test control images of the to-be-tested bacterial strain; calculating a similarity between the testing images and the test control images corresponding to the testing images, to obtain respective similarity test results of the testing images, where each of the similarity test results indicates similarity or dissimilarity; and determining the minimum inhibitory concentration of the to-be-tested antibiotic corresponding to the to-be-tested bacterial strain based on the similarity test results and the preset gradient concentrations.

According to the present disclosure, the similarity between the testing images and the test control images corresponding to the testing images is calculated to obtain the respective similarity test results of the testing images. The similarity between the testing images of antibiotic-bacterial strain and the test control images corresponding to the testing images is calculated, where the antibiotic is of different gradient concentrations, so as to determine the similarity or dissimilarity between the testing images corresponding to the preset gradient concentrations and the test control images. Therefore, the minimum inhibitory concentration of the to-be-tested antibiotic corresponding to the to-be-tested bacterial strain is obtained based on the similarity or dissimilarity corresponding to each of the preset gradient concentrations, rapidly and accurately implementing automatic detection and determination of the minimum inhibitory concentration of the antibiotic, thereby reducing detection cost of the minimum inhibitory concentration of the antibiotic. In addition, the apparatus, device for determining the minimum inhibitory concentration and the computer-readable storage medium are provided according to the present disclosure, which have the same beneficial effects.

### BRIEF DESCRIPTION OF THE DRAWINGS

In order to more clearly illustrate technical solutions in embodiments of the present disclosure or in the conventional technology, the drawings to be used in the description of the embodiments or the conventional technology are briefly described below. Apparently, the drawings in the following description show only embodiments of the present disclosure, and other drawings may be obtained by those skilled in the art from the drawings without any creative work.
FIG. 1 is a flowchart of a method for determining a minimum inhibitory concentration according to an embodiment of the present disclosure;
FIG. 2 is a flowchart of a method for determining a minimum inhibitory concentration according to another embodiment of the present disclosure;
FIG. 3 is a schematic structural diagram of a Siamese neural network model according to the conventional technology;
FIG. 4 is a schematic structural diagram of a preset Siamese neural network model of a method for determining a minimum inhibitory concentration according to another embodiment of the present disclosure;
FIG. 5 is a structural block diagram of an apparatus for determining a minimum inhibitory concentration according to an embodiment of the present disclosure;
FIG. 6 is a schematic structural diagram of a device for determining a minimum inhibitory concentration according to an embodiment of the present disclosure; and
FIG. 7 is a schematic diagram illustrating a specific structure of a device for determining a minimum inhibitory concentration according to an embodiment of the present disclosure.

### DETAILED DESCRIPTION OF THE EMBODIMENTS

In order to make the objective, the technical solutions and advantages of the embodiments of the present disclosure more clear, the technical solutions in the embodiments of the present disclosure are described below in conjunction with the drawings in the embodiments of the present disclosure. Apparently, the embodiments described below are only some embodiments of the present disclosure, rather than all the embodiments. All other embodiments obtained by those skilled in the art based on the embodiments in the present disclosure without any creative work fall within the protection scope of the present disclosure.

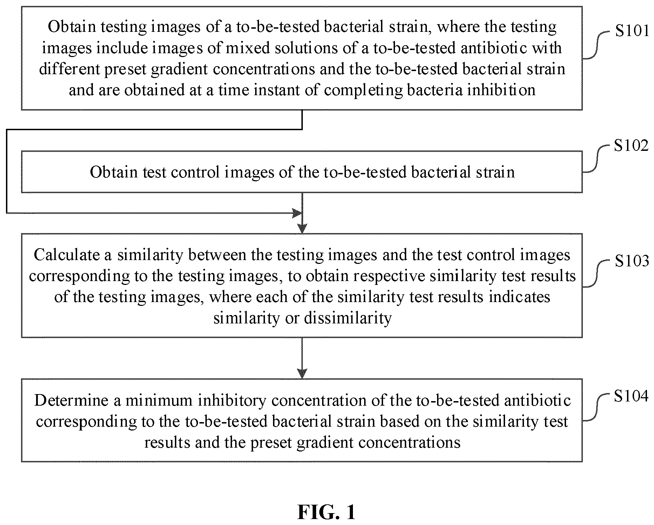

Reference is made to FIG. 1, which is a flowchart of a method for determining a minimum inhibitory concentration according to an embodiment of the present disclosure. The method includes following steps 101 to 104.

In step 101, testing images of a to-be-tested bacterial strain are obtained. The testing images include images of mixed solutions of a to-be-tested antibiotic with different preset gradient concentrations and the to-be-tested bacterial strain and are obtained at a time instant of completing bacteria inhibition.

In the embodiment, the to-be-tested bacterial strain is used to detect and determine a minimum inhibitory concentration of the to-be-tested antibiotic. The to-be-tested antibiotic is used to detect and determine a minimum inhibitory concentration for the to-be-tested bacterial strain. The testing images refer to the images of the mixed solutions of the to-be-tested antibiotic and the to-be-tested bacterial strain, which are used to detect and determine the minimum inhibitory concentration of the to-be-tested antibiotic corresponding to the to-be-tested bacterial strain. That is, the images of mixed solutions obtained at a time instant of completing bacteria inhibition, which is a preset incubation time period after the time instant of completing the preparation of the mixed solutions of the to-be-tested antibiotic with the different preset gradient concentrations and the to-be-tested bacterial strain.

Specific concentration values of each of the preset gradient concentrations may be set by designer according to practical scenarios and user requirements, for example, the concentration gradient of the to-be-tested antibiotic with the preset gradient concentrations may be designed according to rules of the clinical laboratory standard institute (CLSI), as long as a concentration gradient can be formed by all the preset gradient concentrations to facilitate the detection and determination of the minimum inhibitory concentration, which is not limited in the present disclosure.

In an embodiment, the specific content of the testing images of the to-be-tested bacterial strain may be set by the designer according to the practical scenarios and user requirements. For example, the testing images are original images (that is, original testing images) of the mixed solutions of the to-be-tested antibiotic with the preset gradient concentrations and the to-be-tested bacterial strain and are collected by an imaging device. For example, the processor controls an imaging platform to perform lensless imaging (or microscope imaging) on each of microwells after the preset incubation time period following adding the bacterial suspension of the to-be-tested bacterial strain to the microwells of an antimicrobial susceptibility test plate (that is, the time instant of completing bacteria inhibition), to obtain the original testing images (that is, holographic images of the microwells) of the to-be-tested bacterial strain. The testing images may be obtained by performing image processing on the original testing images. For example, for each of the respective original testing images of the microwells, the processor identifies a microwell region on the antimicrobial susceptibility test plate by using the image processing technology, extracts a target region of interest (ROI region) from the microwell region, and inputs the image of the ROI region as the testing image to a corresponding model (such as a preset Siamese neural network model) for detecting the minimum inhibitory concentration. That is, for each microwell of the antimicrobial susceptibility test plate, the processor performs lensless imaging on the microwell after the preset incubation time period following adding the bacterial suspension of the to-be-tested bacterial strain to the microwell, to obtain the original testing image of the to-be-tested bacterial strain. The microwells are provided with the to-be-tested antibiotic with the preset gradient concentrations in one-to-one correspondence. The processor identifies microwell regions in the original testing image, extracts a target region of interest with a preset size from each of the microwell regions, where images of target regions of interest serve as the testing images.

The size, that is, a specification of the preset size, of the ROI region is not limited in the embodiment. For example, the preset size may be 1600 pixels × 1600 pixels, that is, the size of the extracted ROI region may be 1600 pixels × 1600 pixels, because the ROI region of 1600 pixels × 1600 pixels covers most of feature regions of a microwell image (that is, the original testing image). In addition, the processor accurately determines the corresponding relationship between the ROI region and the microwell image, so as to record the type and concentration of the to-be-tested antibiotic and the type of the to-be-tested bacterial strain corresponding to the microwell image.

In step 102, test control images of the to-be-tested bacterial strain are obtained.

In an embodiment, the test control images are used to detect and determine the minimum inhibitory concentration of the to-be-tested antibiotic corresponding to the to-be-tested bacterial strain, that is, the control images used to perform a similarity test with the corresponding testing images.

In an embodiment, the specific content of the test control image may be determined by the designer according to the practical scenarios and user requirements. For example, the test control images may include a preset control image of the test bacterial strain, and the preset control image may be a preset non-inhibition image, that is, an image of a bacterial suspension of the to-be-tested bacterial strain without adding the to-be-tested antibiotic, which is obtained at the time instant of completing the inhibition. The similarity test is performed between the images of the microwells of antibiotic-bacteria combination at the antimicrobial susceptibility test plate and the images of the microwells of the to-be-tested bacterial strain without the antibiotic (a control group) at the antimicrobial susceptibility test plate, where the images are obtained at the time instant of completing bacteria inhibition, and the antibiotic in the antibiotic-bacteria combination are of different gradient concentrations. The preset control image may further be a preset completed-inhibition image, that is, an image of a bacterial suspension of the to-be-tested bacterial strain added with the to-be-tested antibiotic at a preset high concentration, where the image is obtained at the time instant of completing the bacteria inhibition. The similarity test is performed between the images of the microwells of antibiotic-bacteria combination at the antimicrobial susceptibility test plate and the images of the microwells of the to-be-tested bacterial strain being inhibited (a control group) at the antimicrobial susceptibility test plate, where the images are obtained at the time instant of completing bacteria inhibition, and the antibiotic in the antibiotic-bacteria combination are of different gradient concentrations. The test control images may further include the images of the mixed solutions of the to-be-tested antibiotic with the different preset gradient concentrations and the to-be-tested bacterial strain and that are obtained at a time instant of not completing the bacteria inhibition, where the time instant of not completing the bacteria inhibition precedes the time instant of completing the bacteria inhibition, such as the test control images of the to-be-tested bacterial strain obtained by performing imaging (such as the lensless imaging or the microscope imaging) on the microwells in a case that the bacterial suspension of the to-be-tested bacterial strain is added to the microwells of the antimicrobial susceptibility test plate. The test control images may further include a combination of any two or more of the above images. For example, the test control images include the preset non-inhibition image of the to-be-tested bacterial strain, and the images of the mixed solutions of the to-be-tested antibiotic with the different preset gradient concentrations and the to-be-tested bacterial strain and that are obtained at the time instant of not completing bacteria inhibition. Therefore, the accuracy of the detection of the minimum inhibitory concentration is improved through performing the similarity test between the testing images and the multiple corresponding test control images. The specific content of the test control images is not limited in the embodiment.

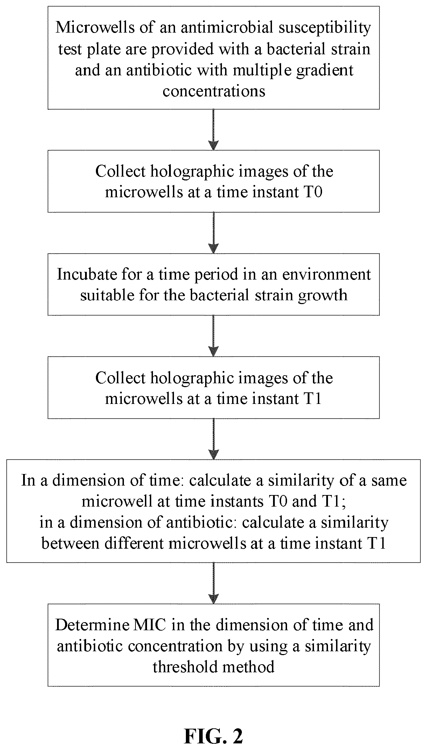

In an embodiment, a manner in which the processor obtains the test control images corresponding to the to-be-tested bacterial strain, may be determined by the designer according to the practical scenarios and user requirements. For example, the processor may directly receive or search for stored test control images corresponding to the to-be-tested bacterial strain, or, the processor may control the process of obtaining the test control images corresponding to the to-be-tested bacterial strain. As shown in FIG. 2, in a case that the test control images include the images of the mixed solution of the to-be-tested antibiotic with the preset gradient concentrations and the to-be-tested bacterial strain and that are obtained at the time instant of not completing bacteria inhibition (time instant T0), the processor performs the lensless imaging (or the microscope imaging) on the microwells at an initial time instant (the time instant T0, that is, the time instant of not completing bacteria inhibition) immediately after the bacterial suspension of the to-be-tested bacterial strain is added to the microwells of the antimicrobial susceptibility test plate, to obtain original control images of the to-be-tested bacterial strain; identifies microwell regions in the obtained original control images; extracts a target control region of interest with a preset size from each of the microwell regions in the original control images, where images of target regions of interest serve as the testing images; performs lensless imaging (or the microscope imaging) on the mircrowells at the time instant (time instant T1) of completing bacteria inhibition after the preset incubation time period following the initial time instant, to obtain the original testing images of the to-be-tested bacterial strain, so as to obtain the testing images of the to-be-tested bacterial strain. In a case that the test control images include the preset non-inhibition image of the to-be-tested bacterial strain, the processor performs the lensless imaging (or the microscope imaging) on the control microwells after the preset incubation time period following adding the bacterial suspension of the to-be-tested bacterial strain to the control microwells (that is, positive control microwells) of the antimicrobial susceptibility test plate, so as to obtain the original control images of the to-be-tested bacterial strain, where the control microwells are not provided with the to-be-tested antibiotic; identifies microwell regions in the original control images; and extracts the target control regions of interest with the preset size from the microwell regions of the original control images, where images of target control regions of interest serve as the testing images.

The antimicrobial susceptibility test plate is a commonly used experimental equipment in antimicrobial susceptibility tests, which consists of multiple regularly arranged microwells. In practice, the antimicrobial susceptibility test plate is of different specifications, generally including a 96-well plate and a 128-well plate. In the implementation, the operator first prepares the bacterial suspension of a sample (that is, the to-be-tested bacterial strain); opens an antibiotic-coated antimicrobial susceptibility test plate, of which the microwells are pre-coated with the to-be-tested antibiotic with different gradient concentrations (that is, the preset gradient concentrations), where the concentration gradients of the antibiotic may be designed according to the rules of the clinical laboratory standard institute; adds the bacterial suspension to the microwells via a sample loading well; and places the antimicrobial susceptibility test plate on the imaging platform upon completing the sample loading. The processor controls the imaging platform to perform lensless imaging on the microwells at an initial time instant immediately after the sample is added (that is, the time instant of not completing inhibition) and a time instant immediately after the preset incubation time period (that is, the time instant of completing bacteria inhibition) and saves the data, so as to obtain the testing images and the test control images of the to-be-tested bacterial strain. Alternatively, the processor performs lensless imaging on the microwells and the control microwells at the time instant immediately after the preset incubation time period and saves the data.

It should be noted that no certain logical sequence exists between step 101 and step 102 in the embodiment. Step 101 may be performed before step 102, step 102 may be performed before step 101, or both two steps may be performed simultaneously, which is not limited in the embodiment.

In step 103, a similarity between the testing images and the test control images corresponding to the testing images is calculated to obtain respective similarity test results of the testing images. Each of the similarity test results indicates similarity or dissimilarity.

It can be understood that in this step, the processor calculates the similarity between the testing images corresponding to the preset gradient concentrations and the test control images corresponding to the testing images, so as to obtain the similarity test result of whether each of the testing images is similar to the corresponding test control image, determining the minimum inhibitory concentration of the to-be-tested antibiotic corresponding to the to-be-tested bacterial strain based on the similarity test results corresponding to the preset gradient concentrations, thus realizing the detection and determination of minimum inhibitory concentration.

In the step, a manner in which the processor calculates the similarity between the testing images and the corresponding test control images to obtain the similarity test results of the testing images may be set by the designer according to the practical scenarios and user requirements, and is not limited herein. For example, in a case that the test control images include images of the mixed solutions of the to-be-tested antibiotic with the different preset gradient concentrations and the to-be-tested bacterial strain and that are obtained at a time instant of not completing bacteria inhibition, the processor calculates the similarity between the testing images and the test control images corresponding the preset gradient concentrations to obtain the similarity test results corresponding to the testing images, that is, the similarity test results of the similarity between each of the testing images corresponding to a preset gradient concentration and the test control image corresponding to the same preset gradient concentration, thereby determining the minimum inhibitory concentration of the antibiotic in a dimension of time. In a case that the test control images include a preset non-inhibition image or a preset completed-inhibition image of the to-be-tested bacterial strain, the processor calculates the similarity between the testing images corresponding to the preset gradient concentrations and the preset non-inhibition image, or calculates the similarity between the testing images corresponding to the preset gradient concentrations and the preset completed-inhibition image, to obtain the respective similarity test results of the testing images, that is, the similarity test result of the similarity between each of the testing images corresponding to a preset gradient concentration and the same preset non-inhibition image, or the similarity test results of the similarity between each of the testing images corresponding to a preset gradient concentration and the same preset completed-inhibition image, thereby determining the minimum inhibitory concentration of the antibiotic in the antibiotic dimension.

As shown in FIG. 2, in the step, the processor may first calculate the similarity between the testing images and the corresponding test control images, and then determine the respective similarity test results of the testing images through a similarity threshold method, that is, the processor determines the similarity test results of the testing images based on the similarity corresponding to the testing images and the preset distance threshold.

In an embodiment, a process in which the processor calculates the similarity between the testing images and the test control images corresponding to the testing images to obtain the similarity test results of the testing images, may be determined by the designer according to the practical scenarios and the user requirements. For example, the processor may calculate the similarity between the testing images and the test control images by using a preset Siamese neural network model, to obtain the similarity test results of the testing images. That is, the processor may calculate the similarity between the testing images and the test control images by using the preset Siamese neural network (also referred to as a dual neural network) model to obtain the similarity test results of the testing images. The processor may also calculate the similarity between the testing images and the test control images by using another algorithm model, which is not limited in the embodiment.

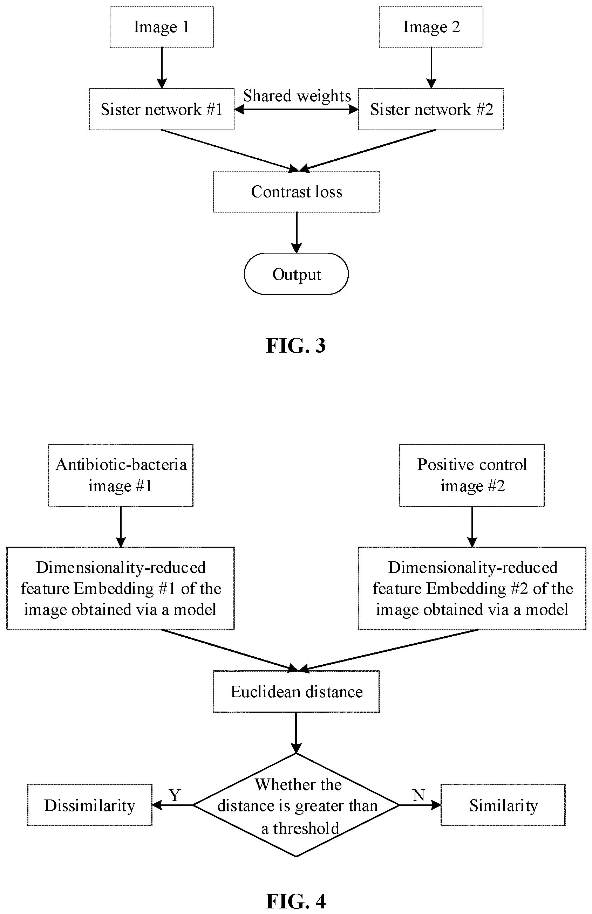

The Siamese neural network is an artificial neural network that uses same weights when operating in tandem on two different input vectors, thereby calculating comparable output vectors. Reference is made to FIG. 3, which shows a structure of the Siamese neural network, and the sister networks (that is, sister network #1 and sister network #2) in the Siamese neural network are identical and share the weights. Sub-network in the Siamese neural network respectively receive different input images and train the neural network by using contrastive loss or triplet loss.

In an embodiment, the sister network in the preset Siamese neural network model is implemented by a feature extraction layer using MobileNet-V2. The MobileNet-V2 is a lightweight neural network and formed by inverted residuals, depthwise separable convolutions and the like. Based on an idea of transfer learning, by means of an MobileNet-V2 classification model trained on an ImageNet (a large visual database for visual object recognition software research) dataset by Google, a final fully-connected layer of the model outputs 1000 nodes, and after being processed through a Softmax (classification network) layer, a probability of each of the 1000 classes of samples in the ImageNet dataset is outputted. In the embodiment, the MobileNet-V2 model is transferred to a problem of determining an image similarity, a global pooling layer and the fully-connected layer is removed from the MobileNet-V2 model and only a fully convolutional feature extraction layer of the model is retained, and then a 1×1 convolutional layer and a global average pooling layer is concatenated to map an image feature to a 128-dimensional space. The entire network is implemented using the fully convolutional operation, which is adaptable to any image size, and the fully connected layer is replaced with a 1×1 convolutional layer, reducing model parameters. The process of a classic residual block is: 1×1 (dimension reduction) -> 3×3 (convolution) -> 1×1 (dimension expansion). The MobileNet-V2 first expands a channel of a feature map through a 1×1 pointwise (PW) convolution operation to increase the number of features, and thus improve accuracy, and then limits extracted features to an input feature dimension via a deep convolutional layer (DP). The sequence of the process of MobileNet-V2 is the reverse of the sequence of the residual block, so it is referred to as inverted residual, including 1×1 (dimension expansion) -> 3×3 (dw-conv+relu) -> 1×1 (dimension reduction + linear transformation). The depthwise separable convolution is an operation that combines the depthwise (DW) convolution and pointwise (PW) convolution, used to extract the feature map. The number of parameters and computational cost of the depthwise separable convolution are relatively low when compared with conventional convolutions.

The sister network in the preset Siamese neural network model may also be another network, such as an improved STN-CBAM-MobileNet model, which is used for extracting features to achieve a better model effect. The improved STN-CBAM-MobileNet model combines MobileNet (a lightweight neural network) with STN (spatial transformation network). An STN module is introduced after the input layer to improve the spatial invariance of the model, effectively enhancing geometric invariance of the convolutional features, thereby improving the robustness of the features. An attention mechanism (Convolutional Block Attention Module, CBAM) is introduced in each Bottleneck module (a convolutional module) to focus more on critical information to the current task, reducing attention to other information, thereby improving the efficiency and accuracy of processing the task.

The sister network in the preset Siamese neural network model may also be a feature extraction layer of another convolutional neural network (CNN), such as MobileNet (depthwise separable convolution) network and VGG network (a classic convolutional neural network), and the like, which is not limited in the embodiment.

In the embodiment, metric learning is used to measure the similarity between samples. The metric learning is considered as similarity learning or distance metric learning. The metric learning includes multiple learning methods such as contrastive loss, margin loss, hinge loss, and triplet loss. The contrast loss is used to increase "inter-class" difference of a classifier, and the triplet loss is an improvement on the contrast loss, reducing the "intra-class" difference as well as increasing the "inter-class" difference. The formula of the contrast loss function is expressed as *L*(*x*₀,*x*_{1,}y) = *y*∥*f*(*x*₀) -*f*(*x*₁)∥*+*(1-*y*)max(0,(m-∥*f*(*x*₀) -*f*(*x*₁)∥)), where *x*₀ and *x*₁ represent two samples, *f*(*x*₀) and *f*(*x*₁) represents sample features extracted by the model; *y* represents a label between the two samples, where *y*=1 indicates that the samples are similar, and *y*=0 indicates that the samples are dissimilar; ∥*f*(*x*₀)-*f*(*x*₁)∥ represents a square of an Euclidean distance; and m (margin) represents a constant hyper-parameter (that is, a preset distance threshold), which is used to set an upper bound (margin square) for the loss, if the distance is greater than or equal to the margin, it is indicated that the model is optimized and the loss is equal to 0, and thus the matching degree of the pair of samples can be effectively expressed.

The formula of the Euclidean distance is expressed as $d \left(x, y\right) = \sqrt{{{\sum }_{i = 1}^{n} \left({x}_{i}-{y}_{i}\right)}^{2}}$, where *x* and *y* represent two points *x*(*x*₁,*x*₂,...,*xₙ*) and *y*(*y*₁,*y*₂*,...,yₙ*) in the *n* dimensional space.

The formula of the triplet loss function may be expressed as *L*(*xₐ,xₚ,xₙ*) = max(0,q+∥*f*(*xₐ*)*-f*(*xₚ*)∥-∥*f*(*xₐ*)*-f*(*xₙ*)∥), where *xₐ* represents an anchor sample, *xₚ* represents a positive sample, and *xₙ* represents a negative sample; q represents a constant hyper-parameter like m in the contrast loss function, which is used to force the model learning, increasing a distance value between different-class samples and reducing a distance value between same-class samples.

The method for determining the minimum inhibitory concentration according to the embodiment may also include a training process of the preset Siamese neural network model. For example, in the embodiment, the loss function used in the training process of the preset Siamese neural network model may be the contrast loss. That is, the processor obtains a training sample set and a training loss function. The training sample set includes a preset number of pairs of training images and image similarity labels corresponding to the preset number of pairs of training images, each of the image similarity labels indicates similarity or dissimilarity, and the training loss function is expressed as $L \left({x}_{0}, {x}_{1}, y\right) = y \left‖f\left({x}_{0}\right)-f\left({x}_{1}\right)\right‖ + \left(1-y\right) max \left(0, \left(m-\left‖f\left({x}_{0}\right)-f\left({x}_{1}\right)\right‖\right)\right) ,$ where *x*₀ and *x*₁ represent two training images in any one pair of the preset number of pairs of training images, *f*(*x*₀) represents a dimensionality-reduced feature of *x*₀ that is extracted by the model, *f*(*x*₁) represents a dimensionality-reduced feature of *x*₁ that is extracted by the model, *y* represents an image similarity label between *x*₀ and *x*₁, m represents the preset distance threshold, and ∥*f*(*x*₀)-f(*x*₁)∥ represents a square of an Euclidean distance between *f*(*x*₀) and *f*(*x*₁). The processor performs iterative training on an initial Siamese neural network model based on the obtained training sample set and the training loss function to obtain the preset Siamese neural network model.

For example, the processor randomly selects two ROI images (that is, the images of the target regions of interest) from the training database and marks the two images with an image similarity label as one pair of the training images, where the image similarity label of the two ROI images being 1 indicates similarity, and the image similarity label of the two ROI images being 0 indicates dissimilarity. The processor selects a preset number of pairs of ROI images to construct the training sample set for training the preset Siamese neural network model, where, the image similarity label of the two ROI images is annotated in advance by using a method combining the broth dilution method and image vision. The processor trains the initial Siamese neural network model based on the constructed training sample set to obtain the preset Siamese neural network model. The training loss function is implemented by the contrast loss, the hyper-parameter (margin, that is, the preset distance threshold) is set to 5, Adam (adaptive moment estimation) is selected as an optimizer, an initial learning rate is set to 0.001, the input images for the training are resized to 256, the batch size is set to 64, the model is iterated for 600 rounds, and l2 regularization (a common type of regularization) is used during the training.

In a case that the test control image includes the preset control image of the to-be-tested bacterial strain(such as the preset non-inhibition images), as shown in FIG. 4 , the processor extracts the dimensionality-reduced features (such as dimensionality-reduced features Embedding #1) of the testing images (antibiotic-bacteria image #1) and the dimensionality-reduced feature (such as dimensionality-reduced feature Embedding #2) of the preset control image (positive control image #2) by using the preset Siamese neural network model, avoiding extracting the dimensionality-reduced features of the preset control image for each time of calculating the Euclidean distance; calculates the Euclidean distances between the dimensionality-reduced features of the testing images and the dimensionality-reduced feature of the preset control image; and determines the similarity test results of the testing images based on the Euclidean distances and the preset distance threshold. That is, the Euclidean distances between the dimensionality-reduced features of the testing images and the dimensionality-reduced feature of the preset control image serve as the similarity used for determination in the similarity threshold method. That is, if the Euclidean distance is greater than the preset distance threshold (margin), the similarity test result is determined as dissimilarity, and if the Euclidean distance is not greater than the preset distance threshold (margin), the similarity test result is determined as similarity.

In step 104, the minimum inhibitory concentration of the to-be-tested antibiotic corresponding to the to-be-tested bacterial strain is determined based on the similarity test results and the preset gradient concentrations.

It can be understood that in the step, the processor determines the minimum inhibitory concentration of the to-be-tested antibiotic corresponding to the to-be-tested bacterial strain based on the respective similarity test results of the testing images and the preset gradient concentrations, thereby realizing the detection and determination of the minimum inhibitory concentration of the antibiotic.

In an embodiment, the manner in which the processor determines the minimum inhibitory concentration of the to-be-tested antibiotic corresponding to the to-be-tested bacterial strain based on the similarity test results and the preset gradient concentrations may be determined by the designer according to the practical scenarios and user requirements. For example, the minimum inhibitory concentration of the to-be-tested antibiotic corresponding to the to-be-tested bacterial strain determined by the processor may be any one of the preset gradient concentrations. For example, in a case that the test control images include the preset non-inhibition image of the to-be-tested bacterial strain, and/or images of the mixed solutions of the to-be-tested antibiotic with the different preset gradient concentrations and the to-be-tested bacterial strain and that are obtained at a time instant of not completing bacteria inhibition, and the similarity test results of the testing images include similarity and dissimilarity, the processor determines a minimum preset gradient concentration of the preset gradient concentrations corresponding to the testing images with similarity test results indicating dissimilarity as the minimum inhibitory concentration, or determines a maximum preset gradient concentration of the preset gradient concentrations corresponding to testing images with similarity test results indicating similarity as the minimum inhibitory concentration. In a case that the test control image includes the preset completed-inhibition image of the to-be-tested bacterial strain, and the similarity test results of the testing images include similarity and dissimilarity, the processor determines a minimum preset gradient concentration of the preset gradient concentrations corresponding to testing images with similarity test results indicating similarity as the minimum inhibitory concentration, or determines a maximum preset gradient concentration of the preset gradient concentrations corresponding to testing images with similarity test results indicating dissimilarity as the minimum inhibitory concentration.

The minimum inhibitory concentration of the to-be-tested antibiotic corresponding to the to-be-tested bacterial strain determined by the processor may not be the preset gradient concentration. In a case that the test control image includes the preset non-inhibition image of the to-be-tested bacterial strain and/or the images of the mixed solutions of the to-be-tested antibiotic with the different preset gradient concentrations and the to-be-tested bacterial strain and that are obtained at a time instant of not completing bacteria inhibition, and the similarity test results of the testing images include similarity and dissimilarity, the processor determines a value (such as an average value) between the minimum preset gradient concentration corresponding to the testing image with similarity test result indicating dissimilarity and the maximum preset gradient concentration corresponding to the testing image with the similarity test result indicating similarity as the minimum inhibitory concentration, which is not limited in the embodiment.

For example, the determination result of the minimum inhibitory concentrations of two commonly used clinical quality control strains (*ATCC25922 Escherichia coli* and *ATCC25923 Staphylococcus aureus)* through the method according to the embodiments validates the feasibility of the method. Preparation of the bacterial strain includes: sub-culturing the strain for the second to fourth generations for standby; selecting colonies to prepare a bacterial suspension with a McFarland 0.5 standard; pre-coating antibiotic in the microwells of the antimicrobial susceptibility test plate (the concentration of the antibiotic is prepared according to CLSI standard); mixing the bacterial suspension with agarose at a concentration of 0.6% to 1.0% and a temperature of 37°C to 40°C in a dilution ratio of 1:300 to 1:500; adding the mixture sample respectively to the microwells by centrifugation; and then placing the antimicrobial susceptibility test plate in an incubator (37±2°C) to incubate for 2 hours to 4 hours. Data collection includes: when the incubation is completed, placing the antimicrobial susceptibility test plate on the lensless imaging platform to take the images of the wells (that is, microwells) and recording the images corresponding to different antibiotic concentrations, where the minimum inhibitory concentration (MIC) determined by using the method in the embodiments is obtained based on the images. As shown in Table 1 and Table 2, it can be seen from the MIC results determined through the method and antimicrobial susceptibility results of the clinical gold standard broth microdilution test (BMD) that the MIC results determined through the method according to the embodiment are more accurate and reliable, and the MIC results are determined in 2 hours to 4 hours through the method according to the embodiment, which is more efficient.

**Table 1 Antimicrobial susceptibility results for ATCC25922 Escherichia coli according to the method and BMD method**

| Antibiotic Name | The method (ug/ml) | BMD(ug/ml) | Remark |
|---|---|---|---|
| Amikacin | 4 | 1-4 | Within the standard range |
| Ampicillin | 4 | 4 | Consistent |
| Cefoxitin | 1 | 2-4 | One gradient tolerance |
| Gentamycin | 0.25 | 0.25-0.5 | Within the standard range |
| Imipenem | 0.12 | 0.06-0.25 | Within the standard range |

**Table 2 Antimicrobial susceptibility results for ATCC25923 Staphylococcus aureus according to the method and BMD method**

| Antibiotic Name | The method (ug/ml) | BMD(ug/ml) | Remark |
|---|---|---|---|
| Erythromycin | 0.5 | 0.25-0.5 | Within the standard range |
| Cefoxitin | 4 | 1-4 | Within the standard range |
| Ampicillin | 1 | 1 | Consistent |

According to the embodiment of the present disclosure, the similarity between the testing images and the test control images corresponding to the testing images is calculated to obtain the respective similarity test results of the testing images. The similarity between the testing images of antibiotic-bacterial strain and the test control images corresponding to the testing images is calculated, where the antibiotic is of different gradient concentrations, so as to determine the similarity or dissimilarity between the testing images corresponding to the preset gradient concentrations and the test control images. Therefore, the minimum inhibitory concentration of the to-be-tested antibiotic corresponding to the to-be-tested bacterial strain is obtained based on the similarity or dissimilarity corresponding to each of the preset gradient concentrations, rapidly and accurately implementing automatic detection and determination of the minimum inhibitory concentration of the antibiotic, thereby reducing detection cost of the minimum inhibitory concentration of the antibiotic.

Corresponding to the above method embodiments, an apparatus for determining a minimum inhibitory concentration is provided according to embodiments of the present disclosure. The apparatus for determining the minimum inhibitory concentration described below and the method for determining the minimum inhibitory concentration described above may be referred to each other.

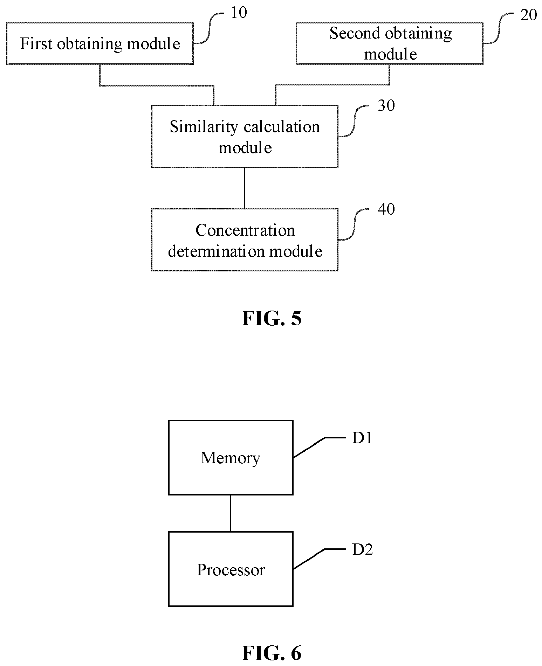

Reference is made to FIG. 5, which is a structural block diagram of an apparatus for determining a minimum inhibitory concentration according to an embodiment of the present disclosure. The apparatus includes a first obtaining module 10, a second obtaining module 20, a similarity calculation module 30, and a concentration determination module 40.

The first obtaining module 10 is configured to obtain testing images of a to-be-tested bacterial strain. The testing images include images of mixed solutions of a to-be-tested antibiotic with different preset gradient concentrations and the to-be-tested bacterial strain and are obtained at a time instant of completing bacteria inhibition.

The second obtaining module 20 is configured to obtain test control images of the to-be-tested bacterial strain.

The similarity calculation module 30 is configured to calculate a similarity between the testing images and the test control images corresponding to the testing images, to obtain respective similarity test results of the testing images. Each of the similarity test results indicates similarity or dissimilarity.

The concentration determination module 40 is configured to determine the minimum inhibitory concentration of the to-be-tested antibiotic corresponding to the to-be-tested bacterial strain based on the similarity test results and the preset gradient concentrations.

In an embodiment, the test control images include a preset control image of the to-be-tested bacterial strain and/or images of the mixed solutions of the to-be-tested antibiotic with the different preset gradient concentrations and the to-be-tested bacterial strain and that are obtained at a time instant of not completing bacteria inhibition, where time instant of not completing bacteria inhibition precedes the time instant of completing bacteria inhibition.

In an embodiment, in a case that the test control images include images of the mixed solutions of the to-be-tested antibiotic with the different preset gradient concentrations and the to-be-tested bacterial strain and that are obtained at the time instant of not completing bacteria inhibition, the similarity calculation module 30 is configured to calculate the similarity between the testing images and the test control images corresponding to the preset gradient concentrations to obtain the respective similarity test results of the testing images.

In an embodiment, the first obtaining module 10 includes a collection sub-module, an identification sub-module, and an extraction sub-module.

The collection sub-module is configured to perform lensless imaging on each of microwells of an antimicrobial susceptibility test plate after a preset incubation time period following adding a bacterial suspension of the to-be-tested bacterial strain to the microwells, to obtain an original testing image of the to-be-tested bacterial strain. The microwells are provided with the to-be-tested antibiotic with the preset gradient concentrations in one-to-one correspondence.

The identification sub-module is configured to identify microwell regions in original testing images.

The extraction sub-module is configured to extract a target region of interest with a preset size from each of the microwell regions. Images of target regions of interest serve as the testing images.

In an embodiment, the minimum inhibitory concentration is any one of the preset gradient concentrations.

In an embodiment, the concentration determination module 40 is further configured to determine, in a case that the similarity test results of the testing images include similarity and dissimilarity, a minimum preset gradient concentration of the preset gradient concentrations corresponding to testing images with similarity test results indicating dissimilarity as the minimum inhibitory concentration.

In an embodiment, the similarity calculation module 30 includes a model calculation sub-module.

The model calculation sub-module is configured to calculate the similarity between the testing images and the test control images corresponding to the testing images by using a preset Siamese neural network model to obtain the respective similarity test results of the testing images.

In an embodiment, sister networks in the preset Siamese neural network model is a feature extraction layer using MobileNet-V2.

In an embodiment, in a case that the test control images include the preset control image of the to-be-tested bacterial strain, the model calculation sub-module includes a feature extraction unit, a distance calculation unit, and a result determination unit.

The feature extraction unit is configured to extract dimensionality-reduced features of the testing images and a dimensionality-reduced feature of the preset control image using the preset Siamese neural network model.

The distance calculation unit is configured to calculate Euclidean distances between the dimensionality-reduced features of the testing images and the dimensionality-reduced feature of the preset control image.

The result determination unit is configured to determine the respective similarity test results of the testing images based on the Euclidean distance and a preset distance threshold.

In an embodiment, the apparatus for determining the minimum inhibitory concentration further includes a model training module.

The model training module is configured to train to obtain the preset Siamese neural network model.

The model training module includes an obtaining sub-module and a training sub-module.

The obtaining sub-module is configured to obtain a training sample set and a training loss function. The training sample set includes a preset number of pairs of training images and image similarity labels corresponding to the preset number of pairs of training images, each of the image similarity labels indicates similarity or dissimilarity, and the training loss function is expressed as *L*(*x*₀,*x*₁*,y*)=*y*∥*f*(*x*₀)*-f*(*x*₁)∥*+*(1*-y*)max(0*,*(*m-*∥*f*(*x*₀)*-f*(*x*₁)∥)), where *x*₀ and *x*₁ represent two training images in any one pair of the preset number of pairs of training images, *f*(*x*₀) represents a dimensionality-reduced feature of *x*₀ that is extracted by the model, *f*(*x*₁) represents a dimensionality-reduced feature of *x*₁ that is extracted by the model, *y* represents an image similarity label between *x*₀ and *x*₁, m represents the preset distance threshold, and ∥*f*(*x*₀)-*f*(*x*₁)∥ represents a square of an Euclidean distance between *f*(*x*₀) and *f*(*x*₁).

The training sub-module is configured to perform iterative training on an initial Siamese neural network model based on the training sample set and the training loss function to obtain the preset Siamese neural network model.

According to the embodiments of the present disclosure, the similarity between the testing images and the test control images corresponding to the testing images is calculated by the similarity calculation module 30 to obtain the respective similarity test results of the testing images. The similarity between the testing images of antibiotic-bacterial strain and the test control images corresponding to the testing images is calculated, where the antibiotic is of different gradient concentrations, so as to determine the similarity or dissimilarity between the testing images corresponding to the preset gradient concentrations and the test control images. Therefore, the minimum inhibitory concentration of the to-be-tested antibiotic corresponding to the to-be-tested bacterial strain is obtained based on the similarity or dissimilarity corresponding to each of the preset gradient concentrations, rapidly and accurately implementing automatic detection and determination of the minimum inhibitory concentration of the antibiotic, thereby reducing detection cost of the minimum inhibitory concentration of the antibiotic.

Corresponding to the above method embodiments, a device for determining a minimum inhibitory concentration is provided according to the embodiment of the present disclosure. The device for determining the minimum inhibitory concentration described below and the method for determining the minimum inhibitory concentration described above may be referred to each other.

Reference is made to FIG. 6, which is a schematic structural diagram of a device for determining a minimum inhibitory concentration according to an embodiment of the present disclosure. The device includes a memory D1 and a processor D2.

The memory D1 stores a computer program.

The processor D2 is configured to is configured to, when executing the computer program, implement the steps of the method for determining the minimum inhibitory concentration provided according to the above method embodiments.

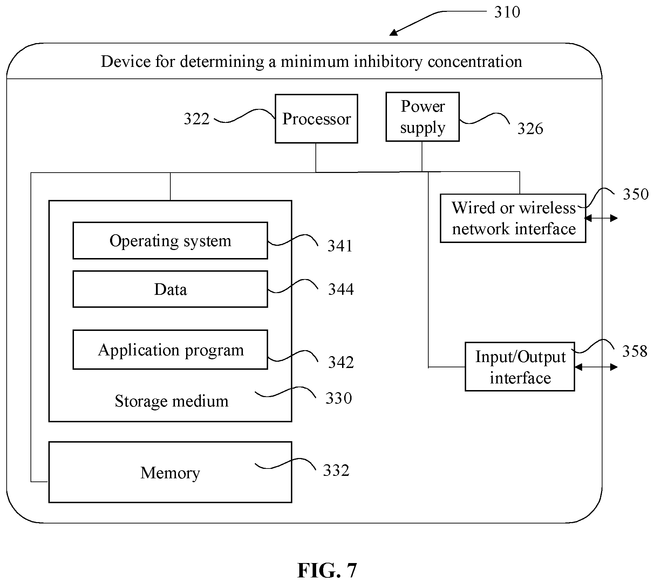

Reference is made to FIG. 7, which is a schematic diagram illustrating a specific structure of a device for determining a minimum inhibitory concentration according to an embodiment of the present disclosure. The device 310 for determining the minimum inhibitory concentration may vary greatly due to different configurations or performances, and may include one or more processors (central processing units, that is, CPUs) 322 (for example, one or more processors) and a memory 332, and one or more storage medias 330 (for example, one or more mass storage devices) storing an application program 342 or data 344. The memory 332 and the storage mediums 330 may perform temporary storage or persistent storage. The programs stored in the storage mediums 330 may include one or more above modules (which are not illustrated in the figure), each module may include a series of instructions operations on the data processing device. Further, the central processing units 322 may be configured to communicate with the storage medium 330, and perform, on the computer device 310, the series of instruction operations stored in the storage medium 330.

The device 310 for determining the minimum inhibitory concentration may further include one or more power supplies 326, one or more wired or wireless network interfaces 350, one or more input/output interfaces 358, and/or one or more operating systems 341. For example, windows ServerTM, Mac OS XTM, UnixTM, LinuxTM, and FreeBSDTM.

The steps of the method for determining the minimum inhibitory concentration described above are implemented by the structure of the device for determining the minimum inhibitory concentration.

Corresponding to the above method embodiments, a computer-readable storage medium is provided according to the embodiment of the present disclosure. The computer-readable storage medium described below and the method for determining the minimum inhibitory concentration described above may be referred to each other.

The computer-readable storage medium stores a computer program. The computer program, when being executed by a processor, implements the steps of the method for determining the minimum inhibitory concentration provided in the above method embodiments.

The computer-readable storage medium may be a readable storage media storing program codes, such as a U disk, a mobile hard disk, a read-only memory (ROM), a random access memory (RAM), a magnetic disc or an optical disc.

The embodiments in the specification are described in a progressive manner. Each of the embodiments mainly focuses on differences from other embodiments, and references can be made to each other for the same or similar parts among the embodiments. Regarding the apparatus, the device and the computer-readable storage medium disclosed in the embodiments, they correspond to the method disclosed in the embodiments and thus the description is simple, and related parts may be seen from the description of the method.

The method, apparatus, and device for determining the minimum inhibitory concentration and the computer-readable storage medium according to the present disclosure are illustrated in detail. The principle and implementation of the present disclosure are illustrated by using specific embodiments herein. The above descriptions of the embodiments are only used to facilitate understanding of the method and the core idea of the present disclosure.

## Claims

1. A computer-implemented method for determining a minimum inhibitory concentration, comprising:
obtaining testing images of a to-be-tested bacterial strain, wherein the testing images comprise images of mixed solutions of a to-be-tested antibiotic with different preset gradient concentrations and the to-be-tested bacterial strain and are obtained at a time instant of completing bacteria inhibition;
obtaining test control images of the to-be-tested bacterial strain;
calculating a similarity between the testing images and the test control images corresponding to the testing images, to obtain respective similarity test results of the testing images, wherein each of the similarity test results indicates similarity or dissimilarity; and
determining the minimum inhibitory concentration of the to-be-tested antibiotic corresponding to the to-be-tested bacterial strain based on the similarity test results and the preset gradient concentrations.

2. The method for determining the minimum inhibitory concentration according to claim 1, wherein
the test control images comprise a preset control image of the to-be-tested bacterial strain, and/or images of the mixed solutions of the to-be-tested antibiotic with the different preset gradient concentrations and the to-be-tested bacterial strain and that are obtained at a time instant of not completing bacteria inhibition, wherein time instant of not completing bacteria inhibition precedes the time instant of completing bacteria inhibition.

3. The method for determining the minimum inhibitory concentration according to claim 2, wherein in response to the test control images comprising images of the mixed solutions of the to-be-tested antibiotic with the different preset gradient concentrations and the to-be-tested bacterial strain and that are obtained at the time instant of not completing bacteria inhibition, the calculating a similarity between the testing images and the test control images corresponding to the testing images, to obtain respective similarity test results of the testing images comprises:
calculating the similarity between the testing images and the test control images corresponding to the preset gradient concentrations to obtain the respective similarity test results of the testing images.

4. The method for determining the minimum inhibitory concentration according to claim 1, wherein the obtaining testing images of a to-be-tested bacterial strain comprises:
performing lensless imaging on each of microwells of an antimicrobial susceptibility test plate after a preset incubation time period following adding a bacterial suspension of the to-be-tested bacterial strain to the microwells, to obtain an original testing image of the to-be-tested bacterial strain, wherein the microwells are provided with the to-be-tested antibiotic with the preset gradient concentrations in one-to-one correspondence;
identifying microwell regions in original testing images; and
extracting a target region of interest with a preset size from each of the microwell regions, wherein an image of the target region of interest serves as the testing image.

5. The method for determining the minimum inhibitory concentration according to claim 1, wherein
the minimum inhibitory concentration is any one of the preset gradient concentrations.

6. The method for determining the minimum inhibitory concentration according to claim 5, wherein the determining the minimum inhibitory concentration of the to-be-tested antibiotic corresponding to the to-be-tested bacterial strain based on the similarity test results and the preset gradient concentrations comprises:
determining, in response to the similarity test results of the testing images comprising similarity and dissimilarity, a minimum preset gradient concentration of the preset gradient concentrations corresponding to testing images with similarity test results indicating dissimilarity as the minimum inhibitory concentration.

7. The method for determining the minimum inhibitory concentration according to any one of claims 1 to 6, wherein the calculating a similarity between the testing images and the test control images corresponding to the testing images, to obtain respective similarity test results of the testing images comprises:
calculating the similarity between the testing images and the test control images corresponding to the testing images by using a preset Siamese neural network model to obtain the respective similarity test results of the testing images.

8. The method for determining the minimum inhibitory concentration according to claim 7, wherein
sister networks in the preset Siamese neural network model are implemented by a feature extraction layer using MobileNet-V2.

9. The method for determining the minimum inhibitory concentration according to claim 7, wherein in response to the test control images comprising the preset control image of the to-be-tested bacterial strain, the calculating the similarity between the testing images and the test control images corresponding to the testing images by using a preset Siamese neural network model to obtain the respective similarity test results of the testing images comprises:
extracting dimensionality-reduced features of the testing images and a dimensionality-reduced feature of the preset control image using the preset Siamese neural network model;
for each of the testing images, calculating an Euclidean distance between the dimensionality-reduced feature of the testing image and the dimensionality-reduced feature of the preset control image; and
determining the respective similarity test results of the testing images based on the Euclidean distances and a preset distance threshold.

10. The method for determining the minimum inhibitory concentration according to claim 9, further comprising a training process of the preset Siamese neural network model,
wherein the training process of the preset Siamese neural network model comprises:
obtaining a training sample set and a training loss function, wherein the training sample set comprises a preset number of pairs of training images and image similarity labels corresponding to the preset number of pairs of training images, each of the image similarity labels indicates similarity or dissimilarity, and the training loss function is expressed as $L \left({x}_{0}, {x}_{1}, y\right) = y \left‖f\left({x}_{0}\right)-f\left({x}_{1}\right)\right‖ + \left(1-y\right) max \left(0, \left(m-\left‖f\left({x}_{0}\right)-f\left({x}_{1}\right)\right‖\right)\right) ,$
where *x*₀ and *x*₁ represent two training images in any one pair of the preset number of pairs of training images, *f*(*x*₀) represents a dimensionality-reduced feature of *x*₀ that is extracted by the model, *f*(*x*₁) represents a dimensionality-reduced feature of *x*₁ that is extracted by the model, *y* represents an image similarity label between *x*₀ and *x*₁, m represents the preset distance threshold, and ∥*f*(*x*₀)-*f*(*x*₁)∥ represents a square of an Euclidean distance between *f*(*x*₀) and *f*(*x*₁); and
performing iterative training on an initial Siamese neural network model based on the training sample set and the training loss function to obtain the preset Siamese neural network model.

11. An apparatus for determining a minimum inhibitory concentration, comprising:
a first obtaining module, configured to obtain testing images of a to-be-tested bacterial strain, wherein the testing images comprise images of mixed solutions of a to-be-tested antibiotic with different preset gradient concentrations and the to-be-tested bacterial strain and are obtained at a time instant of completing bacteria inhibition;
a second obtaining module, configured to obtain test control images of the to-be-tested bacterial strain;
a similarity calculation module, configured to calculate a similarity between the testing images and the test control images corresponding to the testing images, to obtain respective similarity test results of the testing images, wherein each of the similarity test results indicates similarity or dissimilarity; and
a concentration determination module, configured to determine the minimum inhibitory concentration of the to-be-tested antibiotic corresponding to the to-be-tested bacterial strain based on the similarity test results and the preset gradient concentrations.

12. A device for determining a minimum inhibitory concentration, comprising:
a memory, storing a computer program; and
a processor, configured to, when executing the computer program, implement the method for determining the minimum inhibitory concentration according to any one of claims 1 to 10.

13. A computer-readable storage medium, wherein the computer-readable storage medium stores a computer program, and the computer program, when being executed by a processor, implements the method for determining a minimum inhibitory concentration according to any one of claims 1 to 10.

## Patentansprüche

1. Computerimplementiertes Verfahren zur Bestimmung einer minimalen Hemmkonzentration, umfassend
Erfassen von Testbildern eines zu testenden Bakterienstamms, wobei die Testbilder Bilder von Mischlösungen eines zu testierenden Antibiotikums mit unterschiedlichen voreingestellten Gradientenkonzentrationen und des zu testenden Bakterienstamms umfassen und zu einem Zeitpunkt des Abschließens der Bakterienhemmung erfasst werden;
Erfassen von Testkontrollbildern des zu testenden Bakterienstamms;
Berechnen einer Ähnlichkeit zwischen den Testbildern und den den Testbildern entsprechenden Testkontrollbildern, um jeweilige Ähnlichkeitstestergebnisse der Testbilder zu erhalten, wobei jedes der Ähnlichkeitstestergebnisse Ähnlichkeit oder Unähnlichkeit angibt; und
Bestimmen der minimalen Hemmkonzentration des zu testierenden Antibiotikums, die dem zu testenden Bakterienstamm entspricht, basierend auf den Ähnlichkeitstestergebnissen und den voreingestellten Gradientenkonzentrationen.

2. Verfahren zur Bestimmung der minimalen Hemmkonzentration nach Anspruch 1, wobei
die Testkontrollbilder ein voreingestelltes Kontrollbild des zu testenden Bakterienstamms und/oder Bilder der Mischlösungen des zu testierenden Antibiotikums mit den unterschiedlichen voreingestellten Gradientenkonzentrationen und des zu testenden Bakterienstamms umfassen, die zu einem Zeitpunkt des Nichtabschließens der Bakterienhemmung erfasst werden, wobei der Zeitpunkt des Nichtabschließens der Bakterienhemmung dem Zeitpunkt des Abschließens der Bakterienhemmung vorausgeht.

3. Verfahren zur Bestimmung der minimalen Hemmkonzentration nach Anspruch 2, wobei, als Reaktion darauf, dass die Testkontrollbilder Bilder der Mischlösungen des zu testierenden Antibiotikums mit den unterschiedlichen voreingestellten Gradientenkonzentrationen und des zu testenden Bakterienstamms umfassen, die zu dem Zeitpunkt des Nichtabschließens der Bakterienhemmung erfasst werden, das Berechnen einer Ähnlichkeit zwischen den Testbildern und den den Testbildern entsprechenden Testkontrollbildern, um jeweilige Ähnlichkeitstestergebnisse der Testbilder zu erhalten, Folgendes umfasst:
Berechnen der Ähnlichkeit zwischen den Testbildern und den den voreingestellten Gradientenkonzentrationen entsprechenden Testkontrollbildern, um die jeweiligen Ähnlichkeitstestergebnisse der Testbilder zu erhalten.

4. Verfahren zur Bestimmung der minimalen Hemmkonzentration nach Anspruch 1, wobei das Erfassen von Testbildern eines zu testenden Bakterienstamms Folgendes umfasst:
Durchführen einer linsenlosen Bildgebung an jeder der Mikrovertiefungen einer Empfindlichkeitstestplatte für antimikrobielle Mittel nach einer voreingestellten Inkubationszeitdauer nach dem Hinzufügen einer Bakteriensuspension des zu testenden Bakterienstamms zu den Mikrovertiefungen, um ein ursprüngliches Testbild des zu testenden Bakterienstamms zu erhalten, wobei die Mikrovertiefungen mit dem zu testierenden Antibiotikum mit den voreingestellten Gradientenkonzentrationen in Eins-zueins-Entsprechung bereitgestellt sind;
Identifizieren von Mikrovertiefungsbereichen in ursprünglichen Testbildern; und
Extrahieren einer Zielregion von Interesse mit einer voreingestellten Größe aus jedem der Mikrovertiefungsbereiche, wobei ein Bild der Zielregion von Interesse als das Testbild dient.

5. Verfahren zur Bestimmung der minimalen Hemmkonzentration nach Anspruch 1, wobei die minimale Hemmkonzentration eine der voreingestellten Gradientenkonzentrationen ist.

6. Verfahren zur Bestimmung der minimalen Hemmkonzentration nach Anspruch 5, wobei das Bestimmen der minimalen Hemmkonzentration des zu testierenden Antibiotikums, die dem zu testenden Bakterienstamm entspricht, basierend auf den Ähnlichkeitstestergebnissen und den voreingestellten Gradientenkonzentrationen, Folgendes umfasst:
Bestimmen, als Reaktion darauf, dass die Ähnlichkeitstestergebnisse der Testbilder Ähnlichkeit und Unähnlichkeit umfassen, einer minimalen voreingestellten Gradientenkonzentration der voreingestellten Gradientenkonzentrationen, die Testbildern mit Ähnlichkeitstestergebnissen entspricht, die Unähnlichkeit angeben, als minimale Hemmkonzentration.

7. Verfahren zur Bestimmung der minimalen Hemmkonzentration nach einem der Ansprüche 1 bis 6, wobei das Berechnen einer Ähnlichkeit zwischen den Testbildern und den den Testbildern entsprechenden Testkontrollbildern, um jeweilige Ähnlichkeitstestergebnisse der Testbilder zu erhalten, Folgendes umfasst:
Berechnen der Ähnlichkeit zwischen den Testbildern und den den Testbildern entsprechenden Testkontrollbildern mithilfe eines voreingestellten Siamese-Neuronalen-Netzwerkmodells, um die jeweiligen Ähnlichkeitstestergebnisse der Testbilder zu erhalten.

8. Verfahren zur Bestimmung der minimalen Hemmkonzentration nach Anspruch 7, wobei Schwesternnetzwerke in dem voreingestellten Siamese-Neuronalen-Netzwerkmodell durch eine Merkmalsextraktionsschicht mithilfe von MobileNet-V2 implementiert werden.

9. Verfahren zur Bestimmung der minimalen Hemmkonzentration nach Anspruch 7, wobei, als Reaktion darauf, dass die Testkontrollbilder das voreingestellte Kontrollbild des zu testenden Bakterienstamms umfassen, das Berechnen der Ähnlichkeit zwischen den Testbildern und den den Testbildern entsprechenden Testkontrollbildern unter Verwendung eines voreingestellten Siamese-Neuronalen-Netzwerkmodells, um die jeweiligen Ähnlichkeitstestergebnisse der Testbilder zu erhalten, Folgendes umfasst:
Extrahieren dimensionsreduzierter Merkmale der Testbilder und eines dimensionsreduzierten Merkmals des voreingestellten Kontrollbilds unter Verwendung des voreingestellten Siamese-Neuronalen-Netzwerkmodells;
für jedes der Testbilder Berechnen einer euklidischen Distanz zwischen dem dimensionsreduzierten Merkmal des Testbilds und dem dimensionsreduzierten Merkmal des voreingestellten Kontrollbilds; und
Bestimmen der jeweiligen Ähnlichkeitstestergebnisse der Testbilder, basierend auf den euklidischen Distanzen und einem voreingestellten Distanzschwellenwert.

10. Verfahren zur Bestimmung der minimalen Hemmkonzentration nach Anspruch 9, weiter umfassend einen Trainingsprozess des voreingestellten Siamese-Neuronalen-Netzwerkmodells,
wobei der Trainingsprozess des voreingestellten Siamese-Neuronalen-Netzwerkmodells Folgendes umfasst:
Erfassen eines Trainingsstichprobensatzes und einer Trainingsverlustfunktion, wobei der Trainingsstichprobensatz eine voreingestellte Anzahl von Paaren von Trainingsbildern und Bildähnlichkeitskennzeichnungen entsprechend der voreingestellten Anzahl von Paaren von Trainingsbildern umfasst, jede der Bildähnlichkeitskennzeichnungen Ähnlichkeit oder Unähnlichkeit angibt, und die Trainingsverlustfunktion ausgedrückt wird als $L \left({x}_{0}, {x}_{1}, y\right) = y \left‖f\left({x}_{0}\right)-f\left({x}_{1}\right)\right‖ + \left(1-y\right) max \left(0, \left(m-\left‖f\left({x}_{0}\right)-f\left({x}_{1}\right)\right‖\right)\right) ,$
wobei x₀ und x₁ zwei Trainingsbilder in einem Paar der voreingestellten Anzahl von Paaren von Trainingsbildern darstellen, *f*(*x*₀) ein dimensionsreduziertes Merkmal von x₀ darstellt, das durch das Modell extrahiert wird, *f*(*x*₁)ein dimensionsreduziertes Merkmal von x₁ darstellt, das durch das Modell extrahiert wird, y eine Bildähnlichkeitskennzeichnung zwischen x₀ und x₁ darstellt, m den voreingestellten Distanzschwellenwert darstellt, und ∥*f*(*x*₀) -*f*(x₁)∥ ein Quadrat einer euklidischen Distanz zwischen *f*(*x*₀) und *f*(*x*₁)darstellt; und
Durchführen eines iterativen Trainings an einem initialen Siamese-Neuronalen-Netzwerkmodell, basierend auf dem Trainingsstichprobensatz und der Trainingsverlustfunktion, um das voreingestellte Siamese-Neuronale-Netzwerkmodell zu erhalten.

11. Einrichtung zur Bestimmung einer minimalen Hemmkonzentration, umfassend:
ein erstes Erfassungsmodul, ausgebildet zum Erfassen von Testbildern eines zu testenden Bakterienstamms, wobei die Testbilder Bilder von Mischlösungen eines zu testierenden Antibiotikums mit unterschiedlichen voreingestellten Gradientenkonzentrationen und des zu testenden Bakterienstamms umfassen und zu einem Zeitpunkt des Abschließens der Bakterienhemmung erfasst werden;
ein zweites Erfassungsmodul, ausgebildet zum Erfassen von Testkontrollbildern des zu testenden Bakterienstamms;
ein Ähnlichkeitsberechnungsmodul, ausgebildet zum Berechnen einer Ähnlichkeit zwischen den Testbildern und den den Testbildern entsprechenden Testkontrollbildern, um jeweilige Ähnlichkeitstestergebnisse der Testbilder zu erhalten, wobei jedes der Ähnlichkeitstestergebnisse Ähnlichkeit oder Unähnlichkeit angibt; und
ein Konzentrationsbestimmungsmodul, ausgebildet zum Bestimmen der minimalen Hemmkonzentration des zu testierenden Antibiotikums, die dem zu testenden Bakterienstamm entspricht, basierend auf den Ähnlichkeitstestergebnissen und den voreingestellten Gradientenkonzentrationen.

12. Vorrichtung zur Bestimmung einer minimalen Hemmkonzentration, umfassend:
einen Speicher, der ein Computerprogramm speichert; und
einen Prozessor, der ausgebildet ist, beim Ausführen des Computerprogramms das Verfahren zur Bestimmung der minimalen Hemmkonzentration nach einem der Ansprüche 1 bis 10 zu implementieren.

13. Computerlesbares Speichermedium, wobei das computerlesbare Speichermedium ein Computerprogramm speichert und das Computerprogramm, wenn es durch einen Prozessor ausgeführt wird, das Verfahren zur Bestimmung einer minimalen Hemmkonzentration nach einem der Ansprüche 1 bis 10 implementiert.

## Revendications

1. Procédé mis en œuvre par ordinateur pour déterminer une concentration minimale inhibitrice, comprenant
l'obtention d'images de test d'une souche bactérienne à tester, dans lequel les images de test comprennent des images de solutions mélangées d'un antibiotique à tester avec différentes concentrations de gradient prédéfinies et de la souche bactérienne à tester et sont obtenues à un instant d'achèvement d'inhibition de bactéries ;
l'obtention d'images de commande de test de la souche bactérienne à tester ;
le calcul d'une similarité entre les images de test et les images de commande de test correspondant aux images de test, pour obtenir des résultats de test de similarité respectifs des images de test, dans lequel chacun des résultats de test de similarité indique une similarité ou une dissimilarité ; et
la détermination de la concentration minimale inhibitrice de l'antibiotique à tester correspondant à la souche bactérienne à tester sur la base des résultats de test de similarité et des concentrations de gradient prédéfinies.

2. Procédé pour déterminer la concentration minimale inhibitrice selon la revendication 1, dans lequel
les images de commande de test comprennent une image de commande prédéfinie de la souche bactérienne à tester, et/ou des images des solutions mélangées de l'antibiotique à tester avec les différentes concentrations de gradient prédéfinies et de la souche bactérienne à tester et qui sont obtenues à un instant de non-achèvement d'inhibition de bactéries, dans lequel l'instant de non-achèvement d'inhibition de bactéries précède l'instant d'achèvement d'inhibition de bactéries.

3. Procédé pour déterminer la concentration minimale inhibitrice selon la revendication 2, dans lequel, en réponse au fait que les images de commande de test comprennent des images des solutions mélangées de l'antibiotique à tester avec les différentes concentrations de gradient prédéfinies et de la souche bactérienne à tester et qui sont obtenues à l'instant de non-achèvement d'inhibition de bactéries, le calcul d'une similarité entre les images de test et les images de commande de test correspondant aux images de test, pour obtenir des résultats de test de similarité respectifs des images de test, comprend :
le calcul de la similarité entre les images de test et les images de commande de test correspondant aux concentrations de gradient prédéfinies pour obtenir les résultats de test de similarité respectifs des images de test.

4. Procédé pour déterminer la concentration minimale inhibitrice selon la revendication 1, dans lequel l'obtention d'images de test d'une souche bactérienne à tester comprend :
la réalisation d'une imagerie sans lentille sur chacun des micropuits d'une plaque de test de sensibilité aux antimicrobiens après une période de temps d'incubation prédéfinie suivant l'ajout d'une suspension bactérienne de la souche bactérienne à tester aux micropuits, pour obtenir une image de test originale de la souche bactérienne à tester, dans lequel les micropuits sont fournis avec l'antibiotique à tester avec les concentrations de gradient prédéfinies en correspondance biunivoque ;
l'identification de régions de micropuits dans des images de test originales ; et
l'extraction d'une région d'intérêt cible avec une taille prédéfinie à partir de chacune des régions de micropuits, dans lequel une image de la région d'intérêt cible sert d'image de test.

5. Procédé pour déterminer la concentration minimale inhibitrice selon la revendication 1, dans lequel
la concentration minimale inhibitrice est l'une quelconque des concentrations de gradient prédéfinies.

6. Procédé pour déterminer la concentration minimale inhibitrice selon la revendication 5, dans lequel la détermination de la concentration minimale inhibitrice de l'antibiotique à tester correspondant à la souche bactérienne à tester sur la base des résultats de test de similarité et des concentrations de gradient prédéfinies comprend :
la détermination, en réponse au fait que les résultats de test de similarité des images de test comprennent une similarité et une dissimilarité, une concentration de gradient prédéfinie minimale des concentrations de gradient prédéfinies correspondant à des images de test avec des résultats de test de similarité indiquant une dissimilarité comme concentration minimale inhibitrice.

7. Procédé pour déterminer la concentration minimale inhibitrice selon l'une quelconque des revendications 1 à 6, dans lequel le calcul d'une similarité entre les images de test et les images de commande de test correspondant aux images de test, pour obtenir des résultats de test de similarité respectifs des images de test, comprend :
le calcul de la similarité entre les images de test et les images de commande de test correspondant aux images de test en utilisant un modèle de réseau neuronal siamois prédéfini pour obtenir les résultats de test de similarité respectifs des images de test.

8. Procédé pour déterminer la concentration minimale inhibitrice selon la revendication 7, dans lequel
des réseaux jumeaux dans le modèle de réseau neuronal siamois prédéfini sont mis en œuvre par une couche d'extraction de caractéristiques utilisant MobileNetV2.

9. Procédé pour déterminer la concentration minimale inhibitrice selon la revendication 7, dans lequel, en réponse au fait que les images de commande de test comprennent l'image de commande prédéfinie de la souche bactérienne à tester, le calcul de la similarité entre les images de test et les images de commande de test correspondant aux images de test en utilisant un modèle de réseau neuronal siamois prédéfini pour obtenir les résultats de test de similarité respectifs des images de test comprend :
l'extraction de caractéristiques à dimension réduite des images de test et une caractéristique à dimension réduite de l'image de commande prédéfinie en utilisant le modèle de réseau neuronal siamois prédéfini ;
pour chacune des images de test, le calcul d'une distance euclidienne entre la caractéristique à dimension réduite de l'image de test et la caractéristique à dimension réduite de l'image de commande prédéfinie ; et
la détermination des résultats de test de similarité respectifs des images de test sur la base des distances euclidiennes et d'un seuil de distance prédéfini.

10. Procédé pour déterminer la concentration minimale inhibitrice selon la revendication 9, comprenant en outre un processus d'entraînement du modèle de réseau neuronal siamois prédéfini,
dans lequel le processus d'entraînement du modèle de réseau neuronal siamois prédéfini comprend :
l'obtention d'un ensemble d'échantillons d'entraînement et d'une fonction de perte d'entraînement, dans lequel l'ensemble d'échantillons d'entraînement comprend un nombre prédéfini de paires d'images d'entraînement et d'étiquettes de similarité d'image correspondant au nombre prédéfini de paires d'images d'entraînement, chacune des étiquettes de similarité d'image indique une similarité ou une dissimilarité, et la fonction de perte d'entraînement est exprimée comme $L \left({x}_{0}, {x}_{1}, y\right) = y \left‖f\left({x}_{0}\right)-f\left({x}_{1}\right)\right‖ + \left(1-y\right) max \left(0, \left(m-\left‖f\left({x}_{0}\right)-f\left({x}_{1}\right)\right‖\right)\right) ,$
où x₀ et x₁ représentent deux images d'entraînement dans une quelconque paire du nombre prédéfini de paires d'images d'entraînement, *f*(*x*₀) représente une caractéristique à dimension réduite de x₀ qui est extraite par le modèle, f(*x*₁) représente une caractéristique à dimension réduite de x₁ qui est extraite par le modèle, y représente une étiquette de similarité d'image entre x₀ et x₁, m représente le seuil de distance prédéfini, et ∥f(x₀)-f(x₁)∥ représente un carré d'une distance euclidienne entre *f*(*x*₀) et *f*(*x*₁); et
la réalisation d'un entraînement itératif sur un modèle de réseau neuronal siamois initial sur la base de l'ensemble d'échantillons d'entraînement et de la fonction de perte d'entraînement pour obtenir le modèle de réseau neuronal siamois prédéfini.

11. Appareil pour déterminer une concentration minimale inhibitrice, comprenant :
un premier module d'obtention, configuré pour obtenir des images de test d'une souche bactérienne à tester, dans lequel les images de test comprennent des images de solutions mélangées d'un antibiotique à tester avec différentes concentrations de gradient prédéfinies et de la souche bactérienne à tester et sont obtenues à un instant d'achèvement d'inhibition de bactéries ;
un second module d'obtention, configuré pour obtenir des images de commande de test de la souche bactérienne à tester ;
un module de calcul de similarité, configuré pour calculer une similarité entre les images de test et les images de commande de test correspondant aux images de test, pour obtenir des résultats de test de similarité respectifs des images de test, dans lequel chacun des résultats de test de similarité indique une similarité ou une dissimilarité ; et
un module de détermination de concentration, configuré pour déterminer la concentration minimale inhibitrice de l'antibiotique à tester correspondant à la souche bactérienne à tester sur la base des résultats de test de similarité et des concentrations de gradient prédéfinies.

12. Dispositif pour déterminer une concentration minimale inhibitrice, comprenant :
une mémoire, stockant un programme informatique ; et
un processeur, configuré pour, lors de l'exécution du programme informatique, mettre en œuvre le procédé pour déterminer la concentration minimale inhibitrice selon l'une quelconque des revendications 1 à 10.

13. Support de stockage lisible par ordinateur, dans lequel le support de stockage lisible par ordinateur stocke un programme informatique, et le programme informatique, lorsqu'il est exécuté par un processeur, met en œuvre le procédé pour déterminer une concentration minimale inhibitrice selon l'une quelconque des revendications 1 à 10.
